# EUROPEAN PATENT APPLICATION

(11) **EP 1 741 415 A1**
(43) Date of publication of application: **10.01.2007**
(21) Application number: 05106249.5
(22) Date of filing: 08.07.2005
(51) Int. Cl.: A61F 15/00, A61F 13/02, A61F 13/15

(54) **Method and accessories for reducing skin friction during physical exercise**

(71) Applicant: Bio-Racer, 3980 Tessenderlo (BE)
(72) Inventor: Van Parys, Marc, 9000, Gent (BE); De Raeve, Alexandra, 9000, Gent (BE); Godefroidt, Frank, 9000, Gent (BE); Vanstraelen, Raymond, 3780, Tessenderlo (BE); van Osnabrugge, Joost, 3780, Tessenderlo (BE)
(74) Representative: Leherte, Georges M.L.M.

(57) **Abstract**

The invention relates to the use sheets comprising an elastic fabric carrying on one surface a coating of a tacky silicone gel elastomer, as a means for reducing skin friction and/or skin injury during physical exercise such as sports practice, in particular bicycling.

The invention also relates to protective accessories and garments involving such sheets, in particular buttocks protection accessories and garments for cycling practice.

## Description

The invention relates to a method to reduce the consequences of skin friction during physical exercise. Skin frictions resulting from repeated movements, such as occur in work conditions or during sports exercise, like in walking or bicycling, often lead skin injury.

Numerous methods have been considered and/or developed to avoid, alleviate or cure such injuries. These methods may involve a reduction of the pressure exerted by the objects causing a friction, lubricating means for reducing the friction itself, means to heal the injury once it has occurred, etc., etc... All these approaches provide some benefit in specific situations, but also involve specific drawbacks, either inherently or in specific situations.

There remains therefore clearly a need for alternative methods which may be more satisfying for certain individuals, or provide better results in specific situations.

Such a specific situation is for instance the buttocks area / saddle area of sports cyclists.

All "solutions" currently proposed to solve the "saddle sour" problem have shown insufficiently satisfactory in the long run.

Applicants have now conceived a totally new approach to the question, providing a much more satisfying results for the "saddle sour" problem, with interesting potentials in other physical exercise areas.

This new approach according to the present invention involves the use of a sheet comprising an elastic fabric carrying a coating of a tacky silicone gel elastomer, in view of reducing skin friction and/or skin injury during physical exercise.

Such sheets are already known as such, as therapeutic elastic composite materials for treatment and rehabilitation of scar tissue, for instance from the publication WO 02/45698. In this document the presence of a non tacky exterior surface is said to be beneficial to avoid the tendency of clothing adhering thereto and the problem of soiling.

According to WO 02/45698 a specific embodiment of the sheets involving an embedded thermoplastic cushion plate, can be used on parts of the body for preventing bedsores.

This known use of sheets comprising an elastic fabric carrying a coating of a tacky silicone gel elastomer on one surface, involves a totally different approach than for the use according the present invention.

The invention thus relates to the use of a sheet comprising an elastic fabric carrying a coating of a tacky silicone gel elastomer on at least one surface, by applying a piece of such sheet with its tacky silicone coating side (or with one of its tacky silicone coating sides) to a body part so as to reduce skin friction and/or skin injury during physical exercise.

In accordance with the invention, the coating layer of silicone gel on the elastic fabric may take the form of a continuous or discontinuous layer.

A discontinuous layer is however much preferred in order to provide "breathing" properties to the sheet according to the invention.

Such a discontinuous breathing layer may consist of discrete dots of silicone gel material, discrete stripes of silicone gel material, continuous stripes of silicone gel material in one or in several directions (for instance in longitudinal and transverse direction), with a determined thickness of the applied silicone gel material, as defined here below.

According to the invention the most preferred shape of the discontinuous layer of silicone gel material is a regular pattern of silicone gel dots, with dots having a size between 1 and 25 mm, spaced apart from each other in such way that the ratio of "breathing" area (i.e. the area of the pattern fabric not provided with a silicone gel coating) to the area of the coating dots is at least 1 to 2, preferably at least 1 to 1, and most preferably at least 2 to 1.

According to a more specific embodiment of the invention, the piece of sheet is applied with its tacky silicone coating side (or one of its tacky silicone coating sides) to a body part in view of reducing skin friction and/or skin injury during sports practice, whereas according to the most preferred embodiment of the invention, a piece of sheet comprising an elastic fabric carrying a coating of a tacky silicone gel elastomer on at least one surface is adhered, with its tacky coating side (or one of its tacky coating sides) to the buttocks as protection against skin friction and/or skin injury during bicycle practice.

In the embodiment of the invention comprising a tacky coating on both surfaces of the fabric, the sheet is applied with one tacky side to the body, whereas the other tacky side is applied to an intermediate sheet (like for instance a so called "cycling pad" or "shammy" or a sock) serving as a "sliding surface" between the body and object that causes friction (like a bicycle saddle or a shoe part). This intermediate sheet can consist of a separate piece of intermediate sheet, or constitute an appropriate part of a garment (for instance a shorts or a sock).

According to a further feature of the invention the silicone gel elastomer coating results from a two component silicone system for skin adhesion, formulated in such proportions and cured under such conditions to provide the properties appropriate to the application.

These preferred silicone systems are commonly known in the art and commercially available for various applications involving adhesion directly on the skin (such as in particular for breast prosthesis application and for the treatment of burns/fire injuries), and referred to for instance in patent documents US 5,919,476 and EP 0 251 810.

The essence in the context of the present invention is optimal skin friendliness.

Suitable silicone systems are for instance available from WACKER Silicones (in particular under the reference "ELASTOSYL ® P 7010") and from DOW CORNING (in particular under reference "DOW CORNING®3631").

The proportions of the two components of the silicone system will depend on the required tackiness and flexibility of the coating, in relation with the applied curing times.

The tacky silicone gel elastomer coating on the elastic fabric has most suitably a thickness between 50 µ and 1000 p, preferably between 50 µ and 300 p, corresponding a preferred coating layer of approximately 50 - 300 g/m² of silicone coating on the elastic fabric.

According to another preferred feature of the invention, the elastic fabric is a bi-elastic "lycra"-type fabric.

The expression "lycra"-type fabric refers to elastic fabrics comprising a proportion of elastic polyurethane fibres ("lycra" fibres, "elasthan" fibres, etc.) as commonly know in the art.

The essence in this context is the bi-elastic behaviour (same or different elasticity in longitudinal and in transverse directions) of the fabric so that the piece of sheet can optimally follow the body part to which it is applied / adhered (with elasticity up to 150% and more).

For the same reason the fabric is preferably rather thin, for instance a fabric weighing around 210 g/m² (suitable fabrics are those often used nowadays for competition swimsuits and sport cyclist shorts).

The bi-elastic fabric can suitably consist of polyamide and/or polyester fibres together with elastic polyurethane fibres.

The invention also specifically relates to a method for reducing skin friction and/or skin injury during physical exercise by applying a protective sheet to a body part to be protected, by using a sheet comprising an elastic fabric carrying on one surface a coating of a tacky silicone gel elastomer, in the way disclosed above.

The invention furthermore specifically relates to protective sports accessories or garments for reducing skin friction during sports practice, comprising a piece of sheet involving an elastic fabric carrying on one surface a coating of a tacky silicone gel elastomer, provided to be applied with its tacky silicone coating side to a body part.

The most preferred embodiment of this aspect of the invention is a buttocks protection accessory or garment for cycling practice, comprising a piece of sheet involving an elastic fabric carrying on one surface a coating of a tacky silicone gel elastomer, provided to be applied with its tacky silicone coating side to the buttocks of the cyclist.

The protective accessories or garments according to the invention may in particular also involve the preferred features of the silicone gel elastomer coating and/or of the elastic fabric, as referred to above in respect or the new use according to the invention.

The invention has been disclosed here above in respect of its essential features as required for a skilled art person to be able to put the invention to practice.

Many variants to the invention will be readily apparent to the skilled person, beyond the specific features and details set forth in this disclosure, without departing from the basic concept of the invention.

It is thus clear, for instance, that in garments according to the invention only part of the fabric sheet may be coated with silicone gel elastomer, in those areas where adhesion to the body skin (in particular the buttocks) is required.

It is clear also that for certain specific applications other fabrics than those specifically referred to may be appropriate, such as special "cushioning" fabrics not only reducing friction, but also pressure and/or shocks.

## Claims

1. Use of a sheet comprising an elastic fabric carrying a coating of a tacky silicone gel elastomer on at least one surface, **characterised in that** a piece of such sheet is applied with a tacky silicone coating side to a body part in view of reducing skin friction and/or skin injury during physical exercise.

2. Use of a sheet according to claim 1, **characterised in that** a piece of sheet comprising an elastic fabric carrying a coating of a tacky silicone gel elastomer on at least one surface is applied with a tacky silicone coating side to a body part in view of reducing skin friction and/or skin injury during sports practice.

3. Use of a sheet according to claim 1, **characterised in that** a piece of sheet comprising an elastic fabric carrying a coating of a tacky silicone gel elastomer on at least one surface is adhered to the buttocks as protection against skin friction and/or skin injury during bicycle practice.

4. Use of a sheet according to any one of the preceding claims, **characterised in that** said silicone gel elastomer coating results from a two component silicone system for skin adhesion, formulated in such proportions and cured under such conditions to provide the properties appropriate to the application.

5. Use of a sheet according to any one of the preceding claims, **characterised in that** said elastic fabric is a bi-elastic "lycra"-type fabric.

6. Method for reducing skin friction and/or skin injury during physical exercise by applying a protective sheet to a body part to be protected, **characterised in that** a sheet comprising an elastic fabric carrying a coating of a tacky silicone gel elastomer on at least one surface is used in accordance with any one of the preceding claims.

7. Protective sports accessory or garment for reducing skin friction during sports practice, **characterised in that** said accessory or garment comprises a piece of sheet involving an elastic fabric carrying a coating of a tacky silicone gel elastomer on at least one surface, provided to be applied with a tacky silicone coating side to a body part in view of reducing skin friction and/or skin injury during sports practice.

8. Buttocks protection accessory or garment for cycling practice, **characterised in that** said accessory or garment comprises a piece of sheet involving an elastic fabric carrying a coating of a tacky silicone gel elastomer on at least one surface, provided to be applied with its tacky silicone coating side to the buttocks of a cyclist.

9. Protective accessory or garment according to any one of claims 7 and 8, **characterised in that** the silicone gel elastomer coating results from a two component silicone system for skin adhesion, formulated in such proportions and cured under such conditions to provide the properties appropriate to the application.

10. Protective accessory or garment according to any one of claims 7 to 9, **characterised in that** the elastic fabric is a bi-elastic "lycra"-type fabric.
